# EUROPEAN PATENT SPECIFICATION

(11) **EP 1 816 431 B1**
(45) Date of publication and mention of the grant of the patent: **09.03.2016**
(21) Application number: 07000761.2
(22) Date of filing: 16.01.2007
(51) Int. Cl.: G06T 7/00, C12R 1/46, C12N 1/20, C12N 1/04

(54) **Device and method for automatic control of plastic bag handles**
Vorrichtung und Verfahren für die automatische Kontrolle von Plastiktütenhenkeln
Dispositif et procédé de contrôle automatique de poignées de sacs plastiques

(30) Priority: 03.02.2006 IT MI20060184
(43) Date of publication of application: 08.08.2007
(73) Proprietor: Mobert S.r.l., 21053 Castellanza VA (IT)
(72) Inventor: Trezzi, Roberto, 20025 Legnano (MI) (IT)
(74) Representative: Petruzziello, Aldo

(56) References cited:
- EP-A1- 0 433 689
- US-A- 5 854 679

## Description

The present invention refers to a device and to a method for the automatic control of plastic bag handles.

The production line for plastic bags with handles commonly known as shoppers (supermarket bags), comprises a heat-sealing unit for plastic film. A reel of plastic film is loaded on the production line and will be processed to obtain several rows of bags simultaneously (normally 2 - 3 - 4 lanes, but in some cases even 5 or 6 lanes).

The main production steps are:
- unwinding of the film;
- cutting and longitudinal sealing so as to obtain several rows of tubes;
- formation of the side gussets in the bags;
- sealing and the transverse cutting of the bags to the pre-set length;
- gathering the bags in stacks (normally from 50 to 100 pieces);
- conveying the stacks under a die cutting press for the formation of the handles;
- placing the stacks of bags with handles on a conveyor belt for the final packaging, which can be manual or automatic.

Lately, the adoption of servomotors and of electronic components with increasingly high performance have allowed the heat sealing units to reach very high theoretical production speeds (more than 300 cycles a minute). In practical use, however, these production capacities have not been fully utilised, in that a single operator is not able to collect and to package the enormous quantity of bags produced by the machine. Obviously, using more than one operator would cancel out the advantages deriving from the increased productivity.

To overcome this drawback at least in part, production lines with automatic boxing systems have been proposed, which have allowed production speeds to be increased but have created new and more serious problems. In fact, with this solution the quality control on the product can be done only on samples, with the persistent danger that stacks of not perfectly formed bags are also put into the boxes. Therefore the numerous resulting disputes have led the purchasers of heat-sealing units to lack confidence in this solution.

One of the problems most subject to dispute concerns the shape of the handles of the bag. In fact, in order to be accepted, the difference in width between one handle and the other must not be greater than 3-4 mm. Unfortunately, during the manufacture it can happen that the film being processed slips and therefore, when the stack is transferred under the cutting machine, it is shifted sideways with respect to the cutting blade (technically defined as the die cutting blade), with the result that the handles are of different widths from each other.

Another common problem derives from the wear on the striker plate of the die cutting blade. Said blade cuts the bags on a striker plate which may be of PVC, of polypropylene or of other similar materials. During work the plate wears out and at a certain point the blade no longer manages to cut the whole stack completely and the scrap (that is, the part of film between the two handles which must be removed) remains attached to the product.

The operator must therefore intervene to adjust the depth of the cut or to replace the striker plate. If the operator does not notice that the blade is not cutting well, the machine produces a large amount of non-complying bags.

Whilst with the manual collecting system, the operator who boxes the stacks sees if the product is well formed, with the automatic boxing there is no possibility of human checking. Thus with the automatic boxing it is not possible to carry out a constant quality control on the product and in fact controls are always done only on samples.

To overcome this drawback at least in part, a control system is known that involves photoelectric cells disposed after the die cutting machine, to check whether the scrap has been removed. Said photoelectric cells must detect an empty space between the handles after the die cutting. This check.in is perfunctory, however, in that the scrap could be attached only by a small flap and therefore it could not be detected by the photoelectric cell. Furthermore, there is no control as far as the width of the handles is concerned.

US 5 854 679 discloses a system for measuring the characteristics (dimensions, weight, etc.) of a tridimensional object and encloses, *inter alia,* two cameras pointing along two different directions towards the object.

EP 0 433 689 **discloses a bag of plastic film provided with handles.**

Object of the present invention is to overcome the drawbacks of the prior art, by providing an effective automatic checking system that ensures a constant monitoring of the boxed product.

This object is achieved in accordance with the invention with the characteristics listed in appended independent claims 1 and 11.

Advantageous embodiments of the invention are apparent from the dependent claims.

According to the invention, the automatic control system for the handles of plastic bags disposed in stacks comprises a video camera adapted to capture the image of the stack of bags in the portion of the handles and an image processing system which converts the pixels acquired by the video camera into geometric outlines of the handles which can thus be measured to see if the handles (B) of said stack come within a pre-set range of tolerance.

The device according to the invention advantageously further comprises a guide system for the film placed in the bag-forming sealing unit, adapted to reduce the shifting of the film during the manufacture, thus reducing the final waste of product due to out-of-range handles.

This system ensures a precise quality control and thus allows an automatic boxing to be used with consequent savings in time and production costs.

Further characteristics of the invention will be made clearer by the detailed description that follows, referring to a purely exemplifying and therefore non-limiting embodiment thereof, illustrated in the appended drawings, in which:
Figure 1 is a diagrammatic side elevational view illustrating a heat-sealing machine for the formation of plastic bags and the step of picking up a stack of plastic bags;
Figure 1A is a top plan view taken in the direction of the arrow A in Figure 1, in which the stack gripping means are not shown;

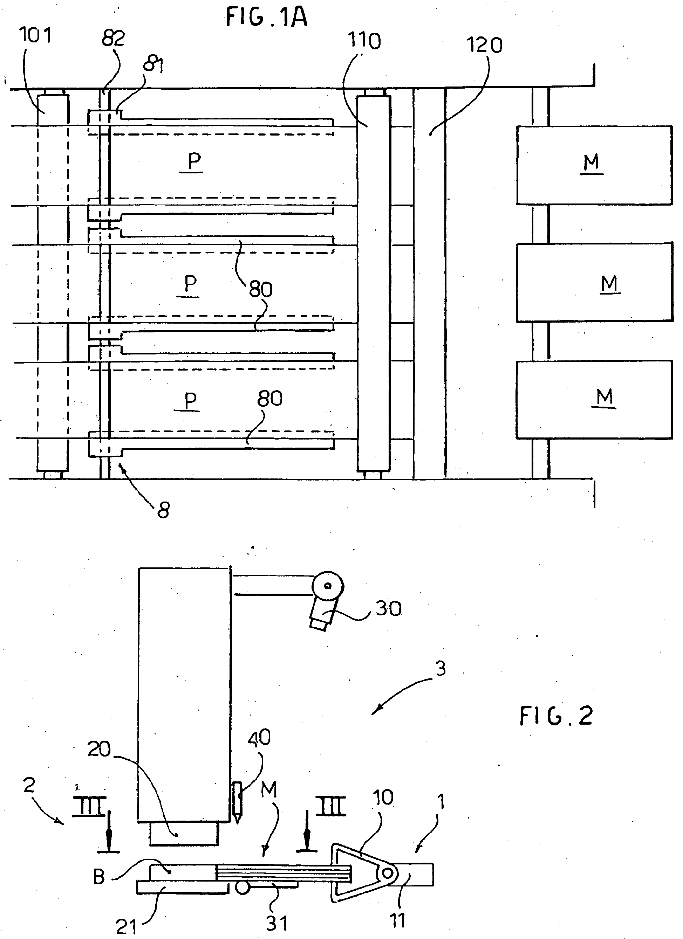

Figure 2 is a diagrammatic side elevational view illustrating the die cutting step of the stack of plastic bags to form the handles;
Figure 3 is a diagrammatic top plan view of a stack of bags, taken in the direction of the arrows III-III in figure 2;
Figure 4 is a diagrammatic side elevational view illustrating the step of controlling the handles of the stack of plastic bags;
Figure 5 is a diagrammatic side elevational view illustrating the step of folding the stack of plastic bags;
Figure 6 is a diagrammatic side elevational view illustrating the step of conveying the stack of plastic bags towards the automatic boxing or towards the waste; and
Figure 7 is a cross sectional view taken along the plane of section VII-VII of Figure 1.

The device and method for automatic control of plastic bag handles according to the invention will be described with the aid of the figures.

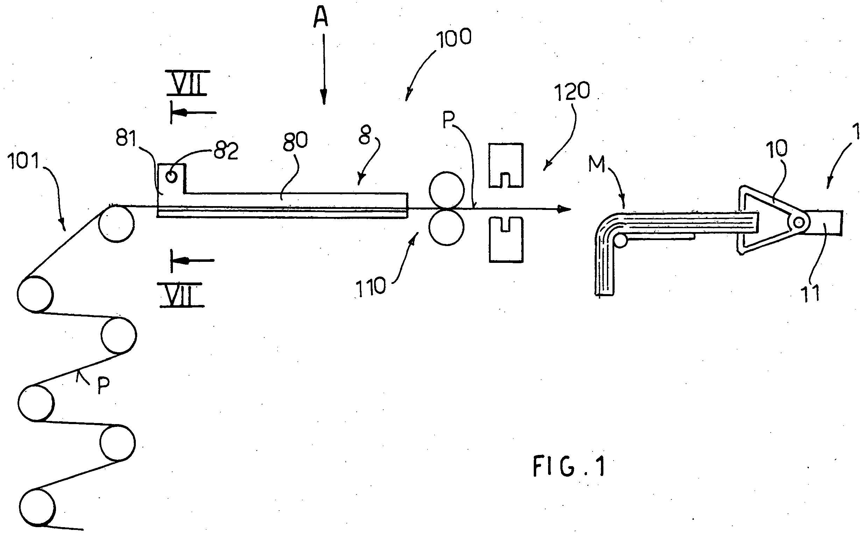

As shown in Figure 1, a heat sealing machine 100, per se known and therefore not described in detail, is used for the production of plastic bags. The bags are obtained starting from a film of plastic material which is cut into longitudinal strips P which run through a roller assembly 101 and are fed in parallel lanes (in Figure 1A three lanes are illustrated).

The films P pass through a calender assembly 110 and a heat-sealing assembly 120 to obtain plastic bags of the desired size which are piled in stacks M. Although reference will be made hereinunder to a single stack M, it must be considered that the production line generally provides for the formation of about 3-5 parallel stacks and that each stack M comprises 50 or 100 bags.

Gripping means 1 are able to pick up and to transport the stack M. The gripping means 1 comprise a series of pincers 10 adapted to clamp one end of the stack M. The pincers 10 are mounted on a horizontally translatable carriage 11.

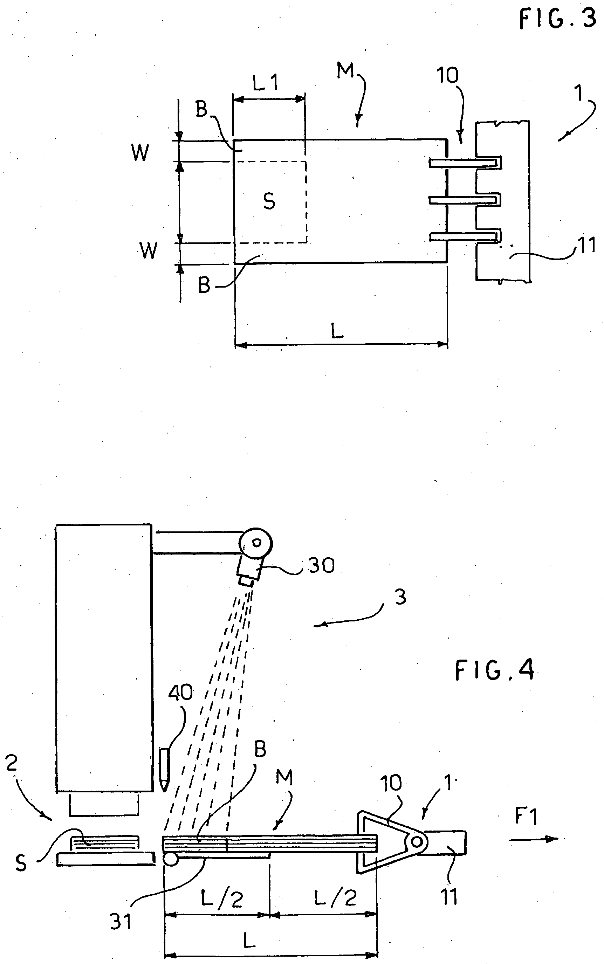

The gripping means 1 convey the stack M towards a die cutting station 2. The die cutting station 2 comprises a die cutting blade 20 that is vertically translatable to about against a striker plate 21. The stack M is disposed between the striking plate 21 and the die cutting blade 20. The die cutting blade 20 is lowered to cut a rectangular portion S (Figure 3) at the end of the stack, so as to form two handles B for each bag of the stack M.

The carriage 11 stops in a pre-set position, so as to obtain a desired length L1 of the handles B, according to the entire original length L of the bag. The width W of the two handles B must be equal, with narrow tolerances which can vary in the range of about 2-3 mm.

As shown in Figure 4, a control station 3 is provided downstream of the cutting station 2. The control station 3 comprises a video camera 30 oriented towards a support 31 disposed downstream of the striker plate 21 of the die cutting station 2. The video camera 30 is able to capture images relating to the stack M which is made to halt on the support 31. The video camera 30 is operatively connected to a computer.

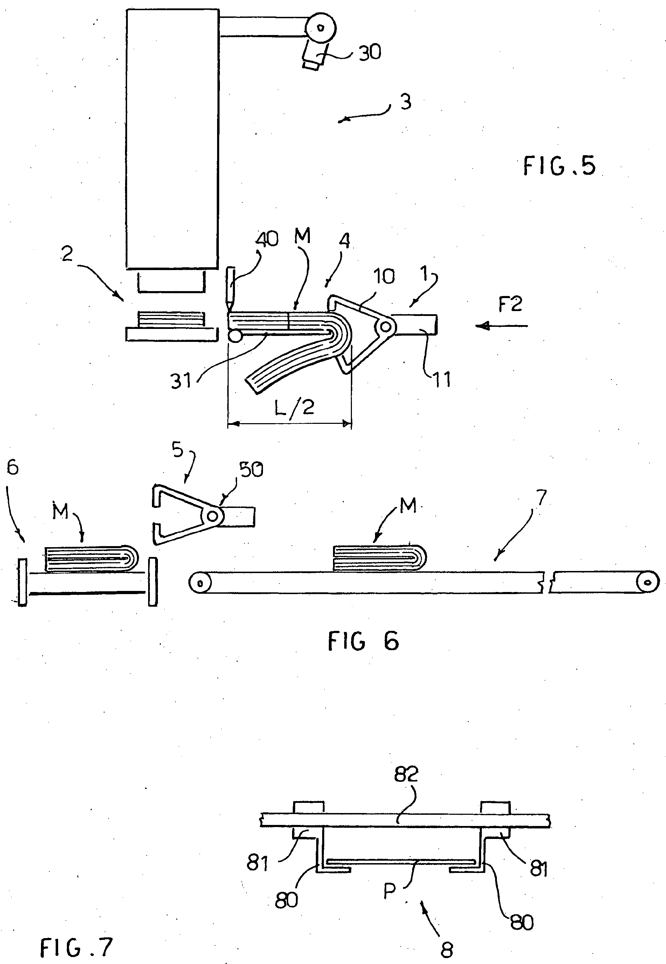

As shown in Figure 5, a folding station 4 adapted to allow the folding of the stack M along a median line can be provided downstream of the control station 3. The folding station 4 comprises a presser 40 adapted to block the stack M on the resting surface of the support 31 along which the folding of the stack takes place.

With reference to Figure 4, the arrow F1 denotes the direction of travel of the carriage 11 to carry the stack M from the die cutting station 2 into the correct position of the control station 3. Once control has taken place, the folding of the stack M is performed.

With reference to Figure 5, the stack blocker 40 comes down, the pincer 10 opens and the carriage 11 is advanced about 100 mm in the direction of the arrow F1. The portion of stack L/2 drops and is folded 90°. The carriage 11 withdraws in the direction of the arrow F2. The pincer closes. As a result the stack M is folded along the support 31, coinciding with the midline of the stack.

For production reasons the stacks may not require folding. In this case the procedure of checking the handles by means of a video camera in any case remains unchanged.

In other applications, the control of the handles can be done even after folding of the stacks M.

As shown in Figure 6, downstream of the folding station 4 a conveying station 5 is provided, which comprises a first transverse conveyor belt 6 to carry the folded stacks M which have passed the control towards the boxing and a second longitudinal conveyor belt 7 to convey the folded stacks which have not passed the control towards waste or a further manual checking. The same gripping means 1 (Figure 4) or further gripping means 50 (Figure 6), substantially the same as the gripping means 1, can be responsible for placing the discarded stacks on the second conveyor belt 7.

Operation of the checking system according to the invention will now be described.

### Video camera calibration step

On installation of the production line or whenever measuring problems arise (for example following a movement or a replacement of the video camera 30), a shape (template) is manually positioned on the support 31 of the control station 3. The template is made of cardboard, foam material, sheet metal or other material and faithfully reproduces the outlines of the stack M of bags that must be checked.

The operator enters into the computer of the viewing system the measurement in millimetres corresponding to the width W of the handle B of the template.

Calibration, which consists in acquiring the image with the video camera 30 and in sending it to the computer, is started by means of the appropriate key. Here a special software which uses advanced, sophisticated mathematical algorithms, faithfully reconstructs the shape of the handles. In practice, the pixels acquired by the viewing system of the video camera 30, corresponding to the outline of the handles B of the template, are converted into millimetres corresponding to the measurement of the width W of the handles B set by the operator.

The calibration operation must be repeated on each manufacturing lane.

### Production step

Before the start of production the operator sets on the computer of the viewing system an error (expressed in millimetres) which can be tolerated on the width W of the handles B. In fact the lack of perfect consistency of size of the stacks of bags must be considered. Since the stack is not a rigid body, it is subject to size variations and the control system must take this into account.

During processing, after each die cut, the stacks M of bags are stopped briefly in the control station 3 and photographed with the video camera 30.

The image of the three or four aligned stacks is then transmitted to the computer which processes the image, converting the pixels corresponding to the outline of each single stack M into millimetres, and thus faithfully reconstructing the outline of the handles B. The software then checks if the width W of the handles B comes within the limits of tolerance set by the operator or not.

The designed software also provides for the possibility that the handles can shift (or slant sideways) during the translation from the die cutting station 2 to the control station 3. In this event too the software is able to carry out a precise measurement of the handles B.

The control system further checks for the possible presence of the scrap S (Figure 3) not perfectly detached from the stack M.

The designed software is able to filter any residue (dirt) left over the time by the plastic film during the die cutting of the handles B.

If from the measurement it proves that the stacks produced come within the set standards of tolerance, said stacks are directed, by means of the first conveyor belt 6, to the automatic boxing. If, on the other hand, the stacks produced do not come within the set standards of tolerance, the boxing station 6 is skipped and the stacks are deposited on the second conveyor 7, where the operator can subsequently decide whether to accept them anyway or to discard them.

With an even more improved system, independently controlled pincers 50 can be provided for each individual lane of stacks M. In this case only the stack M with handles B not complying with the quality standard will be discarded and sent for checking on the longitudinal belt 7.

Normally the waste product due to the fact that the handles B are outside the set tolerance is due to the slipping (lateral shifting) of one or more lanes of the film P (Figure 1A).

In order to reduce this waste, as shown in Figures 1, 1A and 7, a guide system 8 able to guide the film P has been implemented in the bag forming unit of the heat sealer 100. The guide system 8 is disposed before the calenders 110 and the heat-sealing unit 120.

As shown better in Figure 7, the guide system 8 comprises, for each lane, a pair of L-shaped, opposed guides 80. The guides 80 are supported by supports 81 mounted slidably on a transverse shaft 82. The distance between the two guides 80 is adjustable according to the width of the bag to channel the film P, reducing the lateral shifts of the film being processed. The possibilities of discarding stacks because the handles B are outside the pre-set rang are thus reduced.

It can happen that, during the processing, for various reasons (for example imperfections in the reel of plastic film used for processing) the web P being processed in one of the lanes shifts permanently sideways with respect to its ideal working position, causing continuous waste of product because the video camera 3 detects that the width W of the handles B is outside the pre-set range.

In this case the computer connected to the video camera 30 can control per se known film guide devices (one for each processing lane) situated before the heat sealing unit 120, diverting the lane that had shifted back into the correct position by a number of millimetres corresponding to the error detected in the width W of the handles B. Automatic correction of the error is thus obtained.

Numerous changes and modifications of detail within the reach of a person skilled in the art can be made to the present embodiment of the invention without thereby departing from the scope of the invention, as set forth in the appended claims.

## Claims

1. An automatic control device (3) for the handles (B) of plastic bags disposed in stacks (M) comprising
at least one video camera (30) adapted to capture the image of at least one of said stacks (M) of bags, and
processing means adapted to process the pixels of the image captured by the at least one video camera (30) to obtain a faithful geometrical reproduction of the stack; **characterised in that**:
- the at least one video camera (30) is adapted to capture the image of said at least one stack (M) of bags in the position of the handles;
- the processing means are adapted to reconstruct the outline of the handles (B) to measure the width (W) of the handles (B) and to assess whether the measured width (W) of the handles (B) comes within a pre-set range of tolerance,
wherein said automatic control device (3) further includes film guide devices, disposed upstream of the bag forming unit, said film guide devices being controlled by said means for processing the image captured by said video camera (30).

2. A control device (3) according to claim 1, **characterised in that** downstream of said control device (3) a first conveyor (6) to convey the stacks (M) which have passed the control towards the automatic boxing and a second conveyor (7) to convey the stacks (M) which have not passed the control towards the waste or a further manual checking are provided.

3. A control device (3) according to claim 2, **characterised in that** it comprises gripping means (50) controlled by said processing means to pick up the stacks (M) which have not passed the control and to position them on said second conveyor (7).

4. A control device (3) according to any one of the preceding claims, **characterised in that** it comprises a support (31) whereon said stacks of bags are stopped to be filmed by said at least one video camera (30).

5. A control device (3) according to any one of the preceding claims, **characterised in that** it is disposed downstream of a die cutting station (2) of the heat sealing machine for the formation of bags with handles.

6. A control device (3) according to claim 5, **characterised in that** said die cutting station (2) performs the cutting of a scrap (S) from the stacks of bags for the formation of the handles (B) and said control device (3) detects whether said scrap (S) is totally detached from the stacks of bags.

7. A control device (3) according to any one of the preceding claims, **characterised in that** said stacks (M) are disposed side by side on parallel lanes.

8. A control device (3) according to claim 7, **characterised in that** it comprises a single video camera (30) adapted to capture the image of a plurality of stacks side by side on parallel rows.

9. A control device (3) according to claim 7, **characterised in that** it comprises a video camera (30) for each lane of stacks adapted to capture the image of a respective stack.

10. A control device (3) according to any one of the preceding claims, **characterised in that** it comprises a guide system (8) placed in the bag forming unit to channel the film (P) before the sealing unit (120) of the heat sealing machine (100), said guide system (8) reducing the lateral shifting of the web (P) during the processing and consequently limiting the amount of the waste because the product is out of the pre-set range.

11. An automatic control method for the handles (B) of plastic bags disposed in stacks (M), **characterised in that** it comprises the following steps:
- capturing the image of said stacks (M) of bags in the position of the handles (B);
- processing the captured image, converting the pixels of the image into a geometric figure, to measure the width (W) of the handles (B), and
- assessing whether the width of the handles (B) comes within a pre-set range of tolerance.

12. A control method according to claim 11, **characterised in that** it comprises the step of conveying the stacks that have passed the control towards the automatic boxing and of directing the stacks that have not passed the control towards the waste or a further manual checking.

13. A control method according to claim 11 or 12, **characterised in that** before said control step the stacks undergo a die cutting step in which a scrap (S) is cut for the formation of the handles (B).

14. A control method according to any one of claims 11 to 13, **characterised in that** before or after said control step the stacks undergo a folding step in which they are folded along a midline.

15. A control method according to any one of claims 11 to 14, **characterised in that** it comprises the step of guiding the film (P) before the formation of the bag, to reduce lateral shifts of the web (P) during the processing and consequently to limit the amount of the waste because the product is outside the pre-set range.

16. A control method according to any one of claims 11 to 15, wherein provision is made for the film to be guided before the formation of the bag according to processing of the captured image of the stacks of bags.

## Patentansprüche

1. Automatische Kontrollvorrichtung (3) für die Henkel (B) von Plastiktüten, die in Stapeln (M) angeordnet sind, umfassend:
zumindest eine Videokamera (30), die zum Erfassen des Bilds von zumindest einem der Stapel (M) von Tüten geeignet ist, und
Verarbeitungsmittel, die zum Verarbeiten von Pixeln des Bilds, welches durch die zumindest eine Videokamera (30) erfasst wurde, geeignet ist, um eine originalgetreue geometrische Wiedergabe des Stapels zu erzielen;
**dadurch gekennzeichnet, dass**
- die zumindest eine Videokamera (30) zum Erfassen des Bilds des zumindest einen Stapels (M) von Tüten in der Position der Henkel geeignet ist;
- die Verarbeitungsmittel zum Rekonstruieren des Umrisses der Henkel (B) zum Messen der Breite (W) der Henkel (B) und zum Beurteilen, ob die gemessene Breite (W) der Henkel (B) innerhalb eines voreingestellten Toleranzbereichs fällt, geeignet sind,
wobei die automatische Kontrollvorrichtung (3) ferner Filmführungsvorrichtungen enthält, die die stromaufwärts von der Tütenausbildungseinheit angeordnet sind, wobei die Filmführungsvorrichtungen durch die Mittel zum Verarbeiten des Bilds, das durch die Videokamera (30) erfasst ist, kontrolliert werden.

2. Kontrollvorrichtung (3) nach Anspruch 1, **dadurch gekennzeichnet, dass** stromabwärts von der Kontrollvorrichtung (3) ein erster Förderer (6) zum Befördern der Stapel (M), die die Kontrolle passiert haben, zum automatischen Einpacken und ein zweiter Förderer (7) zum Befördern der Stapel (M), die die Kontrolle nicht passiert haben, zum Abfall oder einer weiteren manuellen Prüfung vorgesehen sind.

3. Kontrollvorrichtung (3) nach Anspruch 2, **dadurch gekennzeichnet, dass** es Greifmittel (50), die durch die Verarbeitungsmittel gesteuert sind, zum Aufnehmen der Stapel (M), die die Kontrolle nicht passiert haben, und zum Anordnen derselben auf dem zweiten Förderer (7) umfasst.

4. Kontrollvorrichtung (3) nach einem der vorhergehenden Ansprüche, **dadurch gekennzeichnet, dass** es eine Stütze (31) umfasst, auf der die Tütenstapel angehalten werden, um von der zumindest einen Videokamera (30) gefilmt zu werden.

5. Kontrollvorrichtung (3) nach einem der vorhergehenden Ansprüche, **dadurch gekennzeichnet, dass** sie stromabwärts von einer Stanzstation (2) der Heißsiegelmaschine für die Ausbildung von Tüten mit Henkeln angeordnet ist.

6. Kontrollvorrichtung (3) nach Anspruch 5, **dadurch gekennzeichnet, dass** die Stanzstation (2) das Ausschneiden eines Rests (S) aus den Tütenstapeln für die Ausbildung der Henkel (B) ausführt und die Kontrollvorrichtung (3) erkennt, ob der Rest (S) völlig von den Tütenstapeln losgelöst ist.

7. Kontrollvorrichtung (3) nach einem der vorhergehenden Ansprüche, **dadurch gekennzeichnet, dass** die Stapel (M) nebeneinander auf parallelen Bahnen angeordnet sind.

8. Kontrollvorrichtung (3) nach Anspruch 7, **dadurch gekennzeichnet, dass** sie eine einzige Videokamera (30) umfasst, die zum Erfassen des Bilds von mehreren Stapeln nebeneinander auf parallelen Bahnen geeignet ist.

9. Kontrollvorrichtung (3) nach Anspruch 7, **dadurch gekennzeichnet, dass** sie eine Videokamera (30) für jede Bahn von Stapeln umfasst, die zum Erfassen des Bilds eines jeweiligen Stapels geeignet ist.

10. Kontrollvorrichtung (3) nach einem der vorhergehenden Ansprüche, **dadurch gekennzeichnet, dass** sie ein Führungssystem (8) umfasst, das in der Tütenausbildungseinheit zum Kanalisieren des Films (P) vor der Versiegelungseinheit (120) der Heißsiegelmaschine (100) angeordnet ist, wobei das Führungssystem (8) die seitliche Versetzung des Gewebes (P) während des Verarbeitens reduziert und folglich die Menge des Abfalls, da sich das Produkt außerhalb des voreingestellten Bereichs befindet, begrenzt.

11. Automatisches Kontrollverfahren für die Henkel (B) von Plastiktüten, **dadurch gekennzeichnet, dass** es die folgenden Schritte umfasst:
- Erfassen des Bilds der Stapel (M) von Tüten in der Position der Henkel (B);
- Verarbeiten des erfassten Bilds, Umwandeln der Pixel des Bilds in eine geometrische Figur zum Messen der Breite (W) der Henkel (B) und
- Beurteilen, ob die Breite der Henkel (B) innerhalb eines voreingestellten Toleranzbereichs fällt.

12. Kontrollverfahren nach Anspruch 11, **dadurch gekennzeichnet, dass** es den Schritt des Beförderns der Stapel, die die Kontrolle passiert haben, zum automatischen Einpacken und des Leitens der Stapel, die die Kontrolle nicht passiert haben, zum Abfall oder einer weiteren manuellen Prüfung umfasst.

13. Kontrollverfahren nach einem der Ansprüche 11 oder 12, **dadurch gekennzeichnet, dass** die Stapel vor dem Kontrollschritt einem Stanzschritt unterzogen werden, in dem ein Rest (S) für die Ausbildung der Henkel (B) ausgeschnitten wird.

14. Kontrollverfahren nach einem der Ansprüche 11 bis 13, **dadurch gekennzeichnet, dass** die Stapel vor oder nach dem Kontrollschritt einem Falteschritt unterzogen werden, in dem sie entlang einer Mittellinie gefaltet werden.

15. Kontrollverfahren nach einem der Ansprüche 11 bis 14, **dadurch gekennzeichnet, dass** es den Schritt des Führens des Films (P) vor der Ausbildung der Tüte umfasst, um seitliche Versetzungen des Gewebes (P) während des Verarbeitens zu reduzieren und folglich die Menge des Abfalls, da sich das Produkt außerhalb des voreingestellten Bereichs befindet, zu begrenzen.

16. Kontrollverfahren nach einem der Ansprüche 11 bis 15, **dadurch gekennzeichnet, dass** Maßnahmen getroffen werden, dass der Film vor der Ausbildung der Tüte gemäß dem Verarbeiten des erfassten Bilds der Tütenstapel geführt wird.

## Revendications

1. Dispositif de commande automatique (3) pour les poignées (B) de sacs en plastique disposés en piles (M), comprenant :
au moins une caméra vidéo (30) apte à acquérir l'image d'au moins l'une desdites piles (M) de sacs, et
des moyens de traitement aptes à traiter les pixels de l'image acquise par l'au moins une caméra vidéo (30) pour obtenir une reproduction géométrique fidèle de la pile ;
**caractérisé en ce que** :
- l'au moins une caméra vidéo (30) est apte à acquérir l'image de ladite au moins une pile (M) de sacs à la position des poignées ;
- les moyens de traitement sont aptes à reconstruire le contour des poignées (B) pour mesurer la largeur (W) des poignées (B) et pour évaluer si la largeur mesurée (W) des poignées (B) est conforme à une plage préréglée de tolérance,
dans lequel ledit dispositif de commande automatique (3) comprend en outre des dispositifs de guidage de film, disposés en amont de l'unité de formation de sac, lesdits dispositifs de guidage de film étant commandés par lesdits moyens de traitement de l'image acquise par ladite caméra vidéo (30).

2. Dispositif de commande (3) selon la revendication 1, **caractérisé en ce que**, en aval dudit dispositif de commande (3), il est prévu un premier convoyeur (6) destiné à transporter les piles (M) qui ont passé la commande vers l'emballage automatique et un deuxième convoyeur (7) destiné à transporter les piles (M) qui n'ont pas passé la commande vers les déchets ou un autre contrôle manuel.

3. Dispositif de commande (3) selon la revendication 2, **caractérisé en ce qu'**il comprend des moyens de préhension (50) commandés par lesdits moyens de traitement pour prendre les piles (M) qui n'ont pas passé la commande et pour les positionner sur ledit deuxième convoyeur (7).

4. Dispositif de commande (3) selon l'une quelconque des revendications précédentes, **caractérisé en ce qu'**il comprend un support (31) sur lequel lesdites piles de sacs ne sont plus filmées par ladite au moins une caméra vidéo (30).

5. Dispositif de commande (3) selon l'une quelconque des revendications précédentes, **caractérisé en ce qu'**il est disposé en aval d'une station de découpe (2) de la machine de thermoscellage pour la formation de sacs avec des poignées.

6. Dispositif de commande (3) selon la revendication 5, **caractérisé en ce que** ladite station de découpe (2) effectue la découpe d'un rebut (S) des piles de sacs pour la formation des poignées (B) et ledit dispositif de commande (3) détecte si ledit rebut (S) est totalement détaché des piles de sacs.

7. Dispositif de commande (3) selon l'une quelconque des revendications précédentes, **caractérisé en ce que** lesdites piles (M) sont disposées côte à côte sur des voies parallèles.

8. Dispositif de commande (3) selon la revendication 7, **caractérisé en ce qu'**il comprend une caméra vidéo unique (30) apte à acquérir l'image d'une pluralité de piles côte à côte sur des rangées parallèles.

9. Dispositif de commande (3) selon la revendication 7, **caractérisé en ce qu'**il comprend une caméra vidéo (30) pour chaque voie de piles apte à acquérir l'image d'une pile respective.

10. Dispositif de commande (3) selon l'une quelconque des revendications précédentes, **caractérisé en ce qu'**il comprend un système de guidage (8) placé dans l'unité de formation de sacs pour canaliser le film (P) avant l'unité de scellage (120) de la machine de thermoscellage (100), ledit système de guidage (8) réduisant le déplacement latéral de la bande (P) au cours du traitement et limitant par conséquent la quantité de déchets dus au produit non conforme à la plage préréglée.

11. Procédé de commande automatique pour les poignées (B) de sacs en plastique disposés en piles (M), **caractérisé en ce qu'**il comprend les étapes suivantes :
- l'acquisition de l'image desdites piles (M) de sacs à la position des poignées (B) ;
- le traitement de l'image acquise, en convertissant les pixels de l'image en une figure géométrique, pour mesurer la largeur (W) des poignées (B), et
- l'évaluation si la largeur des poignées (B) est conforme à une plage préréglée de tolérance.

12. Procédé de commande selon la revendication 11, **caractérisé en ce qu'**il comprend l'étape du transport des piles qui ont passé la commande vers l'emballage automatique et l'étape de l'acheminement des piles qui n'ont pas passé la commande vers les déchets ou vers un autre contrôle manuel.

13. Procédé de commande selon la revendication 11 ou 12, **caractérisé en ce que**, avant ladite étape de commande, les piles subissent une étape de découpe à laquelle un rebut (S) est découpé pour la formation des poignées (B).

14. Procédé selon l'une quelconque des revendications 11 à 13, **caractérisé en ce que**, avant ou après ladite étape de commande, les piles subissent une étape de pliage à laquelle elles sont pliées le long d'une ligne médiane.

15. Procédé de commande selon l'une quelconque des revendications 11 à 14, **caractérisé en ce qu'**il comprend l'étape du guidage du film (P) avant la formation du sac, pour réduire des déplacements latéraux de la bande (P) pendant le traitement et pour limiter par conséquent la quantité de déchets dus au produit non conforme à la plage préréglée.

16. Procédé de commande selon l'une quelconque des revendications 11 à 15, dans lequel il est prévu que le film soit guidé avant la formation du sac en fonction du traitement de l'image acquise des piles de sacs.
